Europäisches Patentamt

European Patent Office

Office européen des brevets

(11) Numéro de publication: **0 320 347**
**A1**

# (12) DEMANDE DE BREVET EUROPEEN

(21) Numéro de dépôt: **88403046.1**

(22) Date de dépôt: **02.12.88**

(51) Int. Cl.⁴: **A 61 B 5/05**

(30) Priorité: **08.12.87 FR 8717052**

(43) Date de publication de la demande:
**14.06.89 Bulletin 89/24**

(84) Etats contractants désignés: **DE ES GB IT NL**

(71) Demandeur: **GENERAL ELECTRIC CGR SA.**
**13, Square Max-Hymans**
**F-75015 Paris (FR)**

(72) Inventeur: **Sireul, Jacques**
**CABINET BALLOT-SCHMIT 84, Avenue Kléber**
**F-75116 Paris (FR)**

**Gauthier, René**
**CABINET BALLOT-SCHMIT 84, Avenue Kléber**
**F-75116 Paris (FR)**

**Jacob, Hervé**
**CABINET BALLOT-SCHMIT 84, Avenue Kléber**
**F-75116 Paris (FR)**

(74) Mandataire: **Ballot, Paul Denis Jacques et al**
**Cabinet Ballot-Schmit 84, avenue Kléber**
**F-75116 Paris (FR)**

(54) Dispositif de surveillance du patient dans un appareil d'examen médical.

(57) Un dispositif de surveillance d'un patient dans un appareil d'examen médical, en particulier par résonance magnétique nucléaire, comporte un support (3) sur lequel sont montés une caméra (5) et un miroir (6) renvoyant une image d'une partie (11) du patient vers la caméra, ledit support étant déplaçable par rapport au bâti (1) de l'appareil, mais fixe par rapport au patient.

FIG. 4

EP 0 320 347 A1

**Description**

## DISPOSITIF DE SURVEILLANCE DU PATIENT DANS UN APPAREIL D'EXAMEN MEDICAL

La présente invention concerne un dispositif de surveillance du patient dans un appareil d'examen médical. Elle concerne plus particulièrement un dispositif de surveillance du patient dans un appareil d'imagerie par résonance magnétique nucléaire (en abrége par RMN) ou dans un appareil d'examen de type similaire. En effet, dans un appareil d'imagerie par résonnance magnétique nucléaire, pendant l'examen, le patient se trouve allongé à l'intérieur d'un tunnel formé par les différents aimants créant le champ magnétique, ce tunnel étant lui-même entouré par une cage de Faraday. Le patient se trouve donc dans une ambiance relativement claustrophobique. Pour augmenter le confort du patient, il est donc préférable de ventiler le tunnel d'examen, de l'éclairer et de pouvoir dialoguer avec le patient. De ce fait, le patient est relié à l'opérateur par un système de hauts parleurs et de micros ainsi que par un système manuel de détresse. En tout état de cause, il est nécessaire d'assurer une surveillance constante du patient afin de pouvoir détecter toute anomalie dans son comportement. Pour réaliser cette surveillance, il existe des systèmes de caméra, reliés à un moniteur de surveillance implanté dans le pupitre-contrôle-opérateur. Cette caméra ne peut être positionnée de manière fixe à l'intérieur du tunnel d'examen, car pendant l'examen le patient n'est pas toujours placé au même endroit en fonction des parties de son corps à examiner. Pour pouvoir détecter de manière sûre toute anomalie dans le comportement du patient, les principales zones à observer sont en particulier, la bouche et les yeux. Or, les appareils de surveillance actuellement utilisés ne permettent pas de détecter ces zones quelle que soit la position du patient à l'intérieur du tunnel d'examen.

La présente invention a pour but de remédier à ces inconvénients en proposant un dispositif de surveillance du patient dans un appareil d'examen médical permettant la surveillance du patient quelle que soit sa position à l'intérieur du tunnel d'examen.

La présente invention a aussi pour but de fournir un dispositif de surveillance du patient dans un appareil d'examen médical qui est réglé une fois pour toutes et accepte toutes les configurations d'examen possibles.

En conséquence, la présente invention a pour objet un dispositif de surveillance du patient dans un appareil d'examen médical, caractérisé en ce qu'il comporte un support sur lequel sont montés un caméra et un miroir renvoyant une image d'une partie du patient vers la caméra, ledit support étant déplaçable par rapport au bati de l'appareil mais fixe par rapport au patient.

Selon un mode de réalisation préférentiel, dans le cas d'un appareil d'examen médical comportant un plateau support solidaire du bati de l'appareil sur lequel peut se déplacer un lit porte-patient, le support est monté mobile dans une glissière solidaire du plateau support et est muni d'un moyen d'accrochage au lit porte patient.

De préférence, la glissière est une glissière téléscopique et elle est montée dans une gorge réalisée dans le plateau support. Cette caractéristique permet d'utiliser le dispositif de surveillance même lorsque l'examen à réaliser doit être un examen de la partie inférieure du corps du patient et ceci sans être obligé de retourner le lit pour présenter correctement la zone à examiner. En effet, la zone à examiner doit toujours être centrée au milieu du tunnel d'examen ce qui conduit à des positions relatives du patient par rapport à l'aimant pouvant varier de deux mètres. Avec cette caractéristique, le dispositif de surveillance est toujours correctement cadré et la mise au point réalisée puisque, pendant l'examen, le dispositif de surveillance est solidaire du lit porte patient.

Selon un mode de réalisation préférentiel, cette solidarisation entre le dispositif de surveillance du patient et le lit porte patient est réalisée par un moyen d'accrochage du type fermeture-contact.

Selon une autre caractéristique de la présente invention pour permettre un réglage de la caméra avant l'examen, ladite caméra est fixée sur le support par l'intermédiaire d'un système de réglage trois points. De préférence, l'axe de la caméra fait un angle de 45° par rapport à l'horizontale avec une possibilité de débattement d'environ 10 à 15,° ce qui permet le réglage fin du champ de vision de la caméra/

D'autre part, puisque la caméra est insérée à l'intérieur d'un tunnel d'aimants, pour éviter toute perturbation du champ magnétique la caméra est entièrement blindée.

Selon un autre mode de réalisation de la présente invention, le miroir est constitué par un assemblage de petits miroirs électriquement isolés. Cette constitution spécifique du miroir a pour but d'éviter toute perturbation du champ électromagnétique à l'intérieur du tunnel d'axemen. De préférence, le miroir est réalisé en polycarbonate métallisé. D'autre part, le miroir fait un angle compris entre - 0° et -15° par rapport à l'horizontale, ce qui permet d'obtenir une surveillance correcte des yeux et du visage du patient.

De plus, pour améliorer le confort du patient le dispositif support comporte aussi un miroir secondaire positionné de telle sorte que le patient puisse voir hors de la machine.

D'autres caractéristiques et avantages de la présente invention apparaîtront à la lecture de la description d'un mode de réalisation préférentiel faite ci-après avec référence aux dessins ci-annexés dans lesquels :

- La figure 1 est une vue en perspective d'un dispositif de surveillance du patient placé dans le tunnel d'un appareil d'imagerie par résonance magnétique nucléaire,

- La figure 2 est une vue latérale schématique du dispositif de surveillance de la figure 1 selon deux positions par rapport au plateau support,

- La figure 3 est une vue schématique en élévation latérale d'une caméra utilisée avec le dispositif de surveillance de la présente invention, et

- la figure 4 est une vue en élévation latérale schématique expliquant le fonctionnement du dispositif de surveillance.

Pour simplifier la description dans les figures, les mêmes éléments portent les mêmes références.

D'autre part, la présente invention a été décrite en se référant à un appareil d'imagerie par résonance magnétique nucléaire, toutefois, il est évident pour l'homme de l'art que la présente invention peut être utilisée avec d'autres types d'appareils d'examens médicaux nécessitant une surveillance du patient quelle que soit sa position dans l'appareil d'examen.

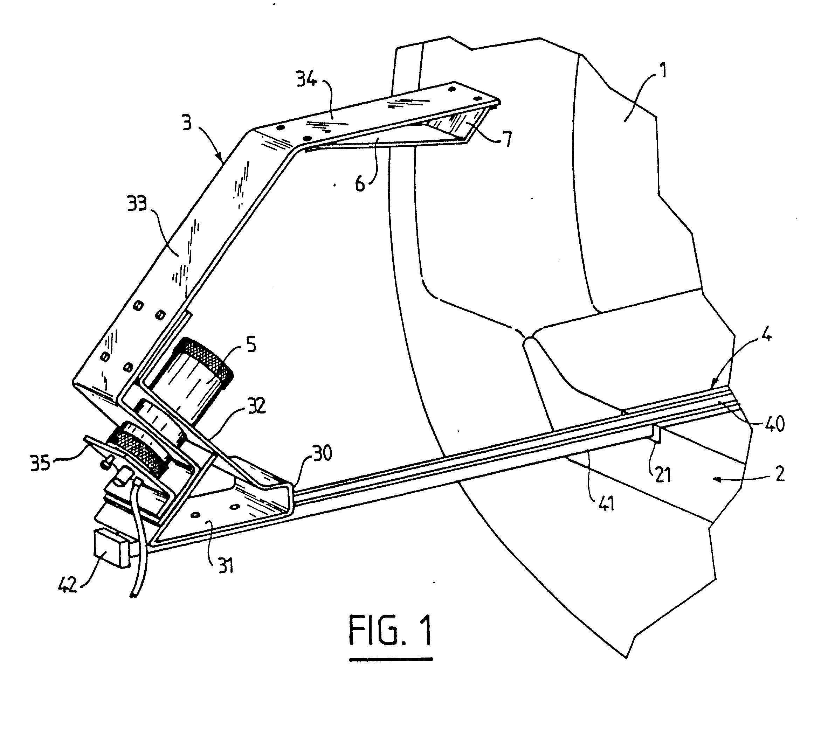

Comme représenté sur la figure 1, un appareil d'imagerie par résonance magnétique nucléaire est constitué essentiellement d'un bati en forme de tunnel 1 destiné à recevoir les aimants nécessaires à la création d'un champ électromagnétique. Sur la partie inférieure du tunnel 1 est monté un plateau support 2. Ce plateau support sert, de manière connue, comme chemin de roulement pour le lit porte-patient non représenté. Conformément à la présente invention, l'appareil d'imagerie par résonance magnétique nucléaire est muni d'un dispositif de surveillance du patient. Ce dispositif de surveillance comporte essentiellement un support 3, une caméra 5 et un miroir 6 renvoyant vers la caméra l'image du patient. Le support 3 est monté de manière à pouvoir se déplacer par rapport au bati de l'appareil, plus particulièrement par rapport au plateau support 2 tout en restant fixe par rapport au patient. Pour ce faire, la partie inférieure du support 3 est munie d'un patin susceptible de coulisser dans un système de glissières 4 monté dans une gorge 21 réalisée dans le plateau support 2. La gorge 21 est réalisée au centre du plateau support 2 dans la partie du tunnel d'examen destinée à recevoir la tête du patient. Le système de glissières 4 utilisé dans la présente invention est un système de glissières télescopiques. Il comporte une première glissière 40 en forme de U à l'intérieur de laquelle peut coulisser une seconde glissière 41 également en forme de U munie d'une gorge centrale recevant le patin du dispositif de surveillance . La seconde glissière 41 comporte à son extrêmité une butée 42 évitant le désengagement du dispositif de surveillance.

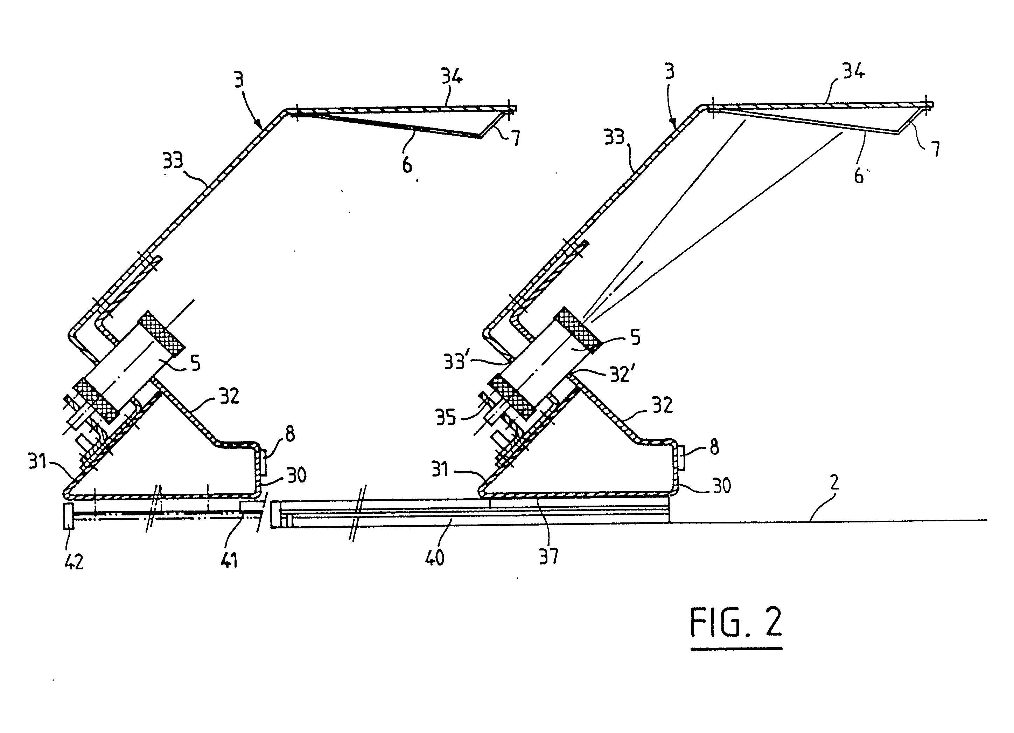

Comme représenté sur les figures 1 et 2, le support 3 du dispositif de surveillance est constitué essentiellement d'une base 30. Cette base réalisée à l'aide de bandes en matière plastique rigide transparrente telle que du "Plexiglass", présente une forme sensiblement triangulaire. Le côté 31 de la base constitue le support de la caméra comme cela sera décrit de manière plus détaillée ci après . Le coté 32 se prolonge vers l'extérieur par une partie en L munie en son milieu d'une ouverture pour le passage de la caméra. D'autre part, le dispositif support comporte un plaque 33 de forme sensiblement en Z dont une extrémité vient se visser sur le coté 31. L'autre extrémité se prolonge par une plaque 34 sensiblement horizontale. La plaque 34 sert de support au miroir 6. Le miroir 6 est constitué par une ensemble de petits miroirs électriquement isolés réalisés, par exemple, en polycarbonate métallisé. Cette constitution particulière du miroir est nécessaire pour éviter de perturber le champ électromagnétique à l'intérieur du tunnel d'examen. Pour avoir une vision correcte de la face du patient, le miroir fait un angle compris entre 0° et - 15° par rapport à l'horizontale. D'autre part, pour augmenter le confort du patient à l'intérieur du tunnel d'examen, le miroir 6 se prolonge par un miroir secondaire 7 fixé entre l'extrémité libre du miroir 6 et la plaque 34. L'orientation du miroir 7 est telle que le malade peut avoir une vision de ce qui se passe à l'extérieur du tunnel d'examen.

Comme représenté sur les figures 1 et 2, le dispositif de surveillance conforme à la présente invention comporte une caméra 5. Cette caméra 5 est fixée par l'intérmédiaire d'un système trois points sur une plaque support 35 solidarisée avec le coté 31 de la base. La plaque 35 présente une forme sensiblement en L . D'autre part, la caméra 5 est insérée dans des ouvertures circulaires pratiquées respectivement dans le coté 32 et le bras 33 du support 3. Les ouvertures circulaires ont un diamètre légèrement supérieur au diamètre de la caméra 5 de manière à permettre un léger débattement angulaire. Il est ainsi possible de régler la déviation angulaire de l'axe de la caméra pour obtenir une très bonne vision de la partie à surveiller du patient. De préférence, l'axe de la caméra forme un angle de 45° par rapport à l'horizontale avec une possibilité de débattement comprise entre 0 et 15°.

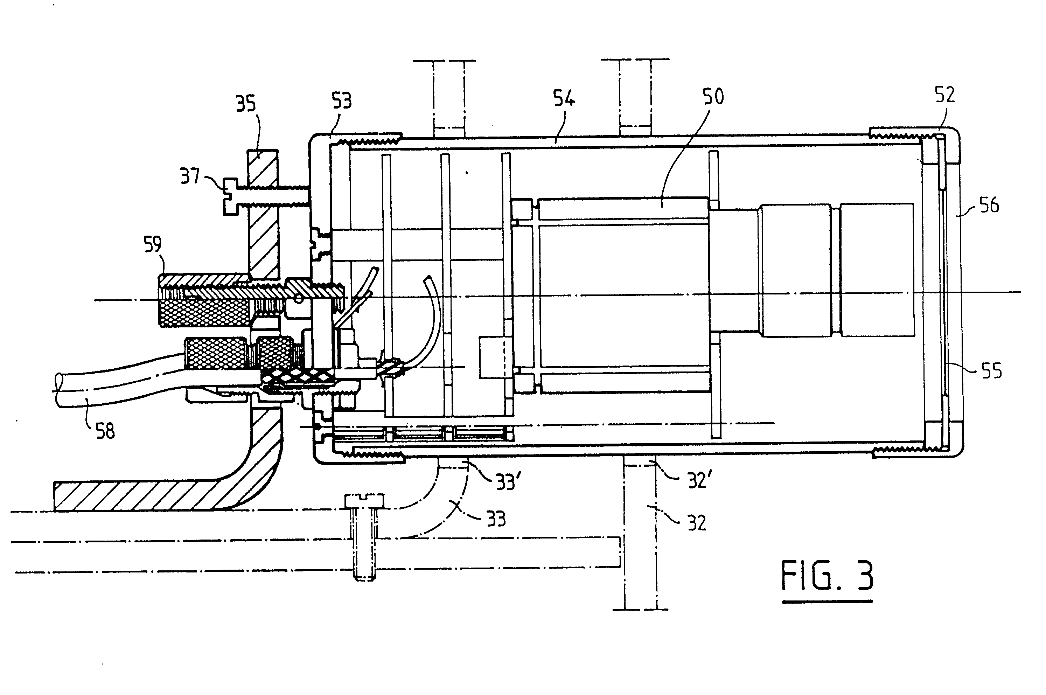

Comme représenté sur la figure 3, la caméra utilisée avec le dispositif de surveillance est une caméra entièrement blindée. En effet, un blindage haute fréquence est nécessaire pour éviter toute perturbation du champ électromagnétique. Ainsi, la caméra 50 est insérée dans un tube 54 réalisé en un matériau amagnétique. A chaque extrémité de ce tube viennent se visser deux capuchons 52 et 53. Le capuchon 52 du coté de l'objectif de la caméra présente un ouverture centrale 56 réalisée en un matériau transparrent et est muni d'une grille 55. Le capuchon 53 présente différentes ouvertures pour le passage des cables de connexion de la caméra avec le système de traitement extérieur. Les cables sont, dans ce cas, constitués par un cable triaxial 58 relié au plan de masse haute fréquence de la cage de Faraday. Ce cable transporte l'alimentation continue de la caméra et le signal vidéo émis par cette dernière.

De préférence, la caméra utilisée dans ce cas est une caméra du type microcaméra avec un capteur CCD (pour charge coupled device) pouvant fonctionner sans altération dans un champ magnétique de 0,5 tesla.

Comme représenté sur les figures 2 et 3, la caméra 5 munie de son blindage est insérée dans les deux ouvertures 32′ et 33′ réalisées respectivement dans le coté 32 et le bras 33 du support 3. La caméra est maintenue en position contre la plaque support 35 par l'intermédiaire de vis 37 formant un système de réglage trois points permettant un léger débattement de l'axe de la caméra. La caméra est bloquée en position par l'intermédiaire d'un écrou 59 se vissant sur en axe fileté solidaire du blindage, l'écrou venant en butée contre la plaque 35.

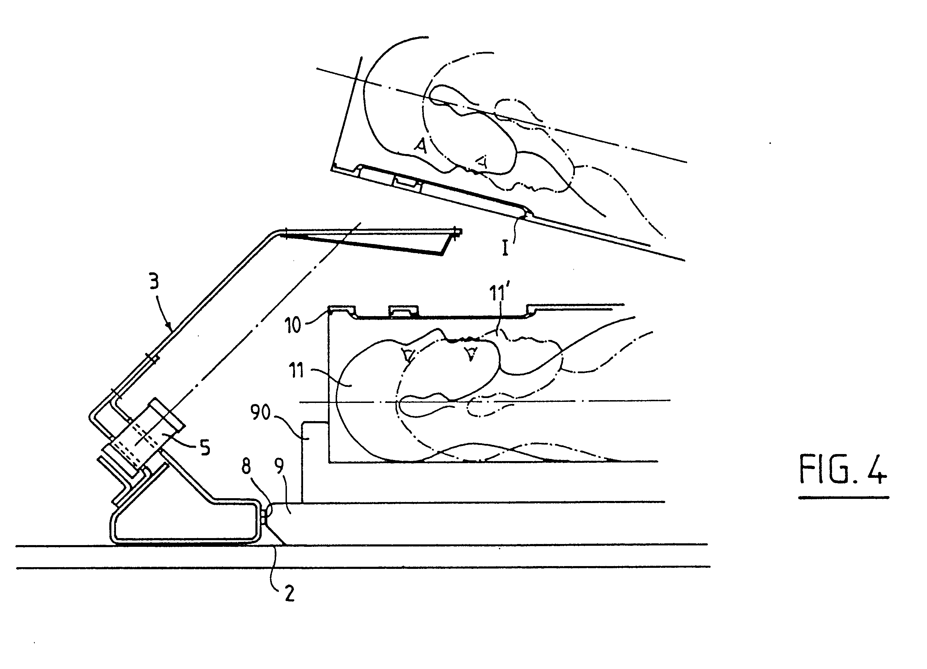

D'autre part, conformément à la présente invention, la base 30 du dispositif de surveillance est muni d'un système d'accrochage 8 du type fermeture-contact, dans le mode réalisation représenté, qui permet l'accrochage du dipositif support sur le lit porte-patient 9 comme représenté sur la figure 4.

On expliquera maintenant en se référant plus particulièrement à la figure 4, le fonctionnement du dispositif de surveillance conforme à la présente invention. Lors d'un examen, le patient est allongé sur le lit porte patient 9, sa tête 11 reposant sur une tétière 90. Le lit porte patient est translaté sur le plateau support 2 et est amené au centre du tunnel d'examen dans une position telle que la tête 11 du patient se trouve en position d'examen. Dans cette position, l'extrémité du lit 9 qui est muni d'un moyen d'accrochage peut s'accrocher avec le dispositif de surveillance par le moyen d'accrochage 8. A ce moment là, le dispositif de surveillance et le lit sont solidarisés l'un avec l'autre et tout mouvement du lit vers le fonds du tunnel d'examen pour permettre l'examen d'autres parties du corps sera suivi par le dispositif de surveillance 3 . En utilisant une caméra dont l'axe de vision forme un angle de 45° par rapport à l'horizontale et un miroir dont la dimension est telle que son exrêmité se trouve sensiblement à l'aplomb du nez du patient lorsque celui-ci est correctement positionné sur la tétière, on obtient un champ de vision de 27° qui permet une surveillance correcte du malade comme représenté sur la figure 4. En effet, dans ce cas, l'image renvoyée vers la caméra par le miroir est l'image virtuelle I. Même dans le cas où l'antenne utilisée est une antenne de tête, c'est -à-dire dans le cas où il existe un champ mort dû à l'armature 10 de l'antenne, la caméra renvoie une image exploitable de la face du patient. Ceci est encore vrai lorsque le patient s'est positionné légèrement en retrait par rapport à la position normale, comme représenté par la silhouette 11' sur la figure 4. Le débattement nécessaire par rapport à la position théorique peut être de plus ou moins 40mm.

Le dispositif de surveillance décrit ci dessus permet donc une surveillance optimale et facile du patient à l'intérieur du tunnel d'examen. Ce dispositif conserve d'autre part sans intervention de l'opérateur, un parfait cadrage de l'image du patient et la distance optimale visage/objectif/caméra et cela quelle que soit la position du lit porte patient dans le tunnel.

Le dispositif de surveillance de la présent invention permet d'avoir une surveillance efficace sans modifier l'image RMN, sans intervention de l'opérateur et cela quel que soit le type d'examen envisagé puisque le dispositif de surveillance du patient est solidarisé avec le lit porte-patient.

## Revendications

1. Dispositif de surveillance du patient dans un tunnel d'un appareil d'examen médical, caractérisé en ce qu'il comporte un support (3) sur lequel sont montés une caméra (15) et un miroir (6) renvoyant une image d'une partie du patient vers la caméra, ledit support étant déplaçable par rapport au bati de l'appareil mais fixe par rapport au patient.

2 - Dispositif selon la revendication 1, caractérisé en ce que, dans le cas d'un appareil d'examen médical comportant un plateau-support (2) solidaire du bati (1) de l'appareil sur lequel peut se déplacer un lit porte-patient, le support (3) est monté mobile dans une glissière (4) solidaire du plateau-support et est muni d'un moyen d'accrochage (8) au lit porte-patient.

3 - Dispositif selon la revendication 2, caractérisé en ce que la glissière (4) est montée dans une gorge (21) réalisée dans le plateau support.

4 - Dispositif selon l'une quelconque des revendications 2 et 3, caractérisé en ce que la glissière est une glissière télescopique (40,41).

5 - Dispositif selon l'une quelconque des revendications 2 à 4, caractérisé en ce que le moyen d'accrochage (8) est du type fermeture-contact.

6 - Dispositif selon l'une quelconque des revendications 1 à 5, caractérisé en ce que la caméra (5) est fixée sur le support par l'intermédiaire d'un système de réglage trois points (37).

7 - Dispositif selon la revendication 6, caractérisé en ce que l'axe de la caméra fait un angle de 45° par rapport à l'horizontal avec possibilité de débattement d'environ 0° à 15°.

8 - Dispositif selon l'une quelconque des revendications 1 à 7 caractérisé en ce que la caméra est blindée.

9 - Dispositif selon l'une quelconque des revendications 1 à 8, caractérisé en ce que le miroir (6) est constitué par un assemblage de petits miroirs électriquement isolés.

10 - Dispositif selon la revendication 9, caractérisé en ce que le miroir est réalisé en polycarbonate métallisé.

11 - Dispositif selon l'une quelconque des revendications 1 à 10, caractérisé en ce que le miroir fait un angle compris entre 0° et - 15° par rapport à l'horizontal.

12 - Dispositif selon l'une quelconque des revendications 1 à 11, caractérisé en ce qu'il comporte de plus un miroir secondaire (7) positionnné pour permettre au patient de voir hors de la machine.

FIG. 1

EP 0 320 347 A1

FIG. 2

EP 0 320 347 A1

FIG. 3

EP 0 320 347 A1

FIG. 4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | US-A-4 650 299  (E.P. STEVENS et al.) * Résumé; colonne 1, lignes 54-62; colonne 2, ligne 61 - colonne 3, ligne 16; colonne 3, ligne 53 - colonne 4, ligne 31; figures 1-3 * | 1,2 | A 61 B    5/05 |
| A | --- | 12 | |
| Y | WO-A-8 404 876  (K.J. VIKTERLÖF et al.) * Résumé; page 2, lignes 5-17; page 5, lignes 21-27; page 6, lignes 3-11; page 6, ligne 34 - page 7, ligne 14; page 8, ligne 27 - page 9, ligne 14; figures 1-4 * | 1,2 | |
| A | EP-A-0 125 808  (L.S. WEINBLATT) * Résumé; page 13, ligne 3 - page 14, ligne 7; figure 4 * | 1 | |
| A | EP-A-0 145 892  (SIEMENS AG) * Résumé; page 1, lignes 11-15; page 2, lignes 17-25; figure 1/1 * | 2-4 | DOMAINES TECHNIQUES RECHERCHES (Int. Cl.4) |
| A | US-A-3 906 953  (R.A. WALLACE et al.) * Résumé; colonne 4, lignes 15-23; figure 1 * | 6 | A 61 B |

Le présent rapport a été établi pour toutes les revendications

| Lieu de la recherche | Date d'achèvement de la recherche | Examinateur |
|---|---|---|
| LA HAYE | 10-03-1989 | RIEB K.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)